# EUROPEAN PATENT APPLICATION

(11) **EP 4 563 184 A1**
(43) Date of publication of application: **04.06.2025**
(21) Application number: 23844954.0
(22) Date of filing: 05.05.2023
(51) Int. Cl.: A61M 37/00

(54) **ADMINISTRATION CONTROL METHOD AND APPARATUS FOR SKIN ADMINISTRATION**

(30) Priority: 27.07.2022 CN 202210891179
(71) Applicant: Beijing Shenzhou Hanfang Pharmaceutical Technology Co., Ltd., Beijing (CN)
(72) Inventor: CHEN, Gang, Beijing 100000 (CN); WANG, Meilin, Beijing 100000 (CN)
(74) Representative: Caspary, Karsten
(86) International application number: PCT/CN2023/092245
(87) International publication number: WO 2024/021739

(57) **Abstract**

The present application provides an administration control method and apparatus for skin administration. The method comprises: S101: determining administration control parameter for skin administration, wherein the control parameter comprises a first control parameter and a second control parameter, one of the first control parameter and the second control parameter is a pressure control parameter, and the other one of the first control parameter and the second control parameter is a temperature control parameter; and S102: controlling a pressure difference for the skin administration by means of the pressure control parameter and the temperature control parameter, the pressure difference being a pressure difference penetrating the skin for administration. The administration apparatus comprises: a pressure assembly and a temperature assembly, wherein the pressure assembly and the temperature assembly control the temperature and the pressure of the skin administration to form the pressure difference for the skin administration; and the administration control method and apparatus form the pressure difference for a drug to penetrate the skin non-invasively at a skin administration site by means of setting the pressure and the temperature to cooperate with each other, so that the problem in the prior art of low administration efficiency during administration is solved and the technical effect of improving the administration efficiency is achieved.

## Description

This application claims priority to the Chinese invention patent application submitted to the China National Intellectual Property Administration on July 27, 2022, with the application number 2022108911799, entitled "Drug Delivery Control Method and Apparatus for Skin Administration", and the entire disclosure thereof is incorporated herein by reference.

### FIELD OF THE DISCLOSURE

This application relates to the field of intelligent medical treatment, and in particular, to a drug delivery control method and apparatus for skin administration.

### BACKGROUND OF THE DISCLOSURE

The drug treatment is a common means for treatment, and the absorption effect of the drug has great influence on the treatment effect. In the prior art, drug administration treatment is carried out in a drug administration mode including oral administration, injection, plaster application, drug steam therapy and the like. The drug administration mode is high in drug requirement, the drug effect is not directly acted on the focus position, the plaster application, the drug steam therapy and the like, the drug is difficult to fully absorb, and the drug administration efficiency is low.

Therefore, the drug delivery apparatus in the prior art has a low drug delivery efficiency.

### SUMMARY OF THE DISCLOSURE

The main purpose of the present application is to provide a drug delivery control method and apparatus for skin administration, so as to solve the technical problem in the prior art of low drug delivery efficiency of a drug delivery apparatus, and achieve the technical effect of improving the drug delivery efficiency of the drug delivery apparatus.

In order to achieve the purpose, the first aspect of the invention provides a drug delivery control method for skin administration The drug delivery control method is controlled through temperature and pressure coupling and is used for controlling a drug to be administered in a manner of noninvasive skin penetration so as to achieve drug delivery treatment, the drug delivery control method includes the following steps:
Determining a drug delivery control parameter for skin administration, wherein the control parameter includes a first control parameter and a second control parameter, one of the first control parameter and the second control parameter is a pressure control parameter, and the other one of the first control parameter and the second control parameter is a temperature control parameter;
Controlling the pressure difference of skin administration through the pressure control parameter and the temperature control parameter, wherein the pressure difference is the pressure difference for feeding the skin through the skin.

In some embodiments of this application, the drug delivery control method is applied to a drug delivery apparatus provided with an auxiliary drug delivery unit and a direct drug delivery unit, the drug delivery control method includes:
Identifying the pressure control parameter to obtain a first pressure control parameter and a second pressure control parameter;
Controlling the auxiliary drug delivery unit to generate a first pressure according to the first pressure control parameter, wherein the first pressure is used for fixing the drug delivery apparatus to the skin and forming a closed drug delivery area;
Controlling the direct drug delivery unit to generate a second pressure according to the second pressure control parameter, the first pressure being less than the second pressure; and
Controlling the direct drug delivery unit to generate a drug delivery temperature according to the temperature control parameter, wherein the drug delivery temperature, the first pressure and the second pressure form a pressure difference of the skin drug delivery in the closed drug delivery area.

In some embodiments of this application, the administration temperature, the first pressure and the second pressure form a pressure difference of the skin administration in the closed drug delivery area includes:
Obtaining drug delivery pressure difference data, wherein the drug delivery pressure difference data is data used for representing the pressure difference in the closed drug delivery area;
Judging whether the drug delivery pressure difference data satisfies a preset drug delivery pressure difference range or not,
If not, generating an updated pressure control parameter, wherein the updated first pressure data and the updated second pressure data are determined according to the updated pressure control parameter until the drug delivery pressure difference data satisfies the preset drug delivery pressure difference range;
If so, the administration is carried out in the closed drug delivery area.

In some embodiments of the present application, the pressure difference between the administration temperature, the first pressure and the second pressure forming the skin administration in the sealed administration area includes:
Obtaining drug delivery pressure difference data, wherein the drug delivery pressure difference data is data used for representing a pressure difference in a closed area;
Determining whether the drug delivery pressure difference data satisfies a preset drug delivery pressure difference range;
If not, generating an update temperature control parameter, wherein the update temperature is determined according to the temperature control parameter until the drug delivery pressure difference satisfies a preset drug delivery pressure difference range;
If so, administration is carried out in the sealed administration area.

In some embodiments of this application, determining administration control parameter for skin administration includes:
Determining first control parameter change data, wherein the first control parameter change data is parameter change data used for representing a first control parameter; and
Determining a parameter change of the second control parameter according to the first control parameter change data to obtain second control parameter change data.

In some embodiments of this application, it is determined that administration control parameter for skin administration includes:
Determining administration feature data, wherein the administration feature data includes drug feature data and administration position feature data;
Matching a drug delivery control model corresponding to the drug feature data and the administration position feature data in a preset drug delivery database, wherein the drug delivery control model is a model used for representing a change of the first control parameter and the second control parameter in a drug delivery time; and
Determining a change in the first control parameter and the second control parameter according to a drug delivery control model.

In some embodiments of the present application, the pressure difference of skin administration is controlled by means of the pressure control parameter and the temperature control parameter, comprising:
Determining the pressure difference of the skin administration according to the first pressure, the second pressure and the administration temperature, wherein the expression of the administration pressure difference is: ci = f (va, vb,T), wherein ci is the pressure difference, va is the first pressure in the direct drug delivery unit, vb is the second pressure of the auxiliary drug delivery unit, and T is the administration temperature of the direct drug delivery unit.

In some embodiments of the invention, a temperature detection element is arranged in the administration apparatus and is used for detecting the temperature in the administration area so as to control matching administration , the administration control method includes the following steps:
Acquiring administration temperature detection data, wherein the administration temperature detection data is temperature data in an administration area;
The administration detection data is determined on the basis of a preset temperature range,
If the detection temperature does not satisfy a preset temperature range, generating a temperature control parameter. According to a second aspect of the present application, provided is a drug delivery control apparatus for skin administration, which is controlled through temperature and pressure coupling and is used for controlling a drug to be administered in a manner of non-invasive skin penetration so as to achieve drug delivery treatment the drug delivery control apparatus includes:
   A parameter determination module used for determining an administration control parameter for skin administration, wherein the control parameter includes a first control parameter and a second control parameter, a first parameter in the first control parameter and the second control parameter is a pressure control parameter, and the other parameter in the first control parameter and the second control parameter is a temperature control parameter;
   A pressure difference module which is used for controlling the pressure difference of skin administration through the pressure control parameter and the temperature control parameter, wherein the pressure difference is the pressure difference for feeding the skin through the skin.

In some embodiments of this application, the drug delivery control apparatus is applied to a drug delivery apparatus provided with an auxiliary drug delivery unit and a direct drug delivery unit, and the drug delivery control apparatus includes:
An identification module for identifying the pressure control parameter to obtain a first pressure control parameter and a second pressure control parameter;
A first pressure module used for controlling the auxiliary drug delivery unit to generate first pressure according to the first pressure control parameter, wherein the first pressure is used for fixing the drug delivery apparatus to the skin and forming a closed drug delivery area;
A second pressure module for controlling the direct drug delivery unit to generate a second pressure according to the second pressure control parameter, the first pressure being less than the second pressure;
A temperature control module used for controlling the direct drug delivery unit to generate a drug administration temperature according to the temperature control parameter;
A pressure difference administration module which is used for forming a pressure difference of skin administration in the closed administration area according to the administration temperature, the first pressure and the second pressure.

In some embodiments of the present application, the pressure difference administration module comprises:
a pressure difference acquisition module which is used for acquiring drug delivery pressure difference data, wherein the drug delivery pressure difference data is data used for representing the pressure difference in the closed drug delivery region;
a pressure difference judgment module which is used for judging whether the drug delivery pressure difference data meets a preset drug delivery pressure difference range or not,
a pressure difference control module, if not, generating an updated pressure control parameter, wherein the updated first pressure data and the updated second pressure data are determined according to the updated pressure control parameter until the administration pressure difference data meets the preset drug delivery pressure difference range; and
if so, administration is carried out in the sealed administration area.

In some embodiments of the present application, the pressure difference administration module includes:
a pressure difference acquisition module which is used for acquiring drug delivery pressure difference data, wherein the drug delivery pressure difference data is data used for representing the pressure difference in the closed drug delivery region;
a pressure difference judgment module which is used for judging whether the drug delivery pressure difference data meets a preset drug delivery pressure difference range or not,
a temperature control module, if not, generating an update temperature control parameter, wherein the update temperature is determined according to the temperature control parameter until the drug delivery pressure difference satisfies a preset drug delivery pressure difference range;
if so, administration is carried out in the sealed administration area.

in some embodiments of the present application, the parameter determination module includes:
a first control parameter module, used for determining first control parameter change data, wherein the first control parameter change data is parameter change data used for representing a first control parameter; and
a second control parameter module, used for determining a parameter change of the second control parameter according to the first control parameter change data, so as to obtain second control parameter change data.

In some embodiments of the present application, the parameter determination module includes:
a drug delivery data acquisition module used for determining drug delivery feature data, wherein the drug delivery feature data includes drug feature data and drug delivery position feature data;
a model matching module which is used for matching a drug delivery control model corresponding to the drug feature data and the drug delivery position feature data in a preset drug delivery database, wherein the drug delivery control model is a model used for representing the first control difference and the second control polygonum change in the drug delivery time; and
a drug delivery parameter determination module, configured to determine, according to a drug delivery control model, a change in the first control parameter and the second control parameter.

In some embodiments of the present application,
a pressure difference module which is used for determining the pressure difference of skin administration according to the first pressure, the second pressure and the administration temperature, wherein the expression of the administration pressure difference is ci = f (va, vb, T), ci is the pressure difference, va is the first pressure in the direct drug delivery unit, vb is the second pressure of the auxiliary drug delivery unit, and T is the administration temperature of the direct drug delivery unit.

In some embodiments of the invention, a temperature detection element is arranged in the drug delivery apparatus and is used for detecting the temperature in the drug delivery region so as to control the matching drug delivery, and the drug delivery control apparatus includes:
a drug delivery temperature detection module used for obtaining drug administration temperature detection data, wherein the drug delivery temperature detection data is temperature data in a drug delivery region;
a drug delivery temperature judgment module which is used for judging the administration detection data based on a preset temperature range,
the administration temperature control module generates temperature control parameters if the detection temperature does not meet a preset temperature range.

According to a third aspect of the present application, provided is a drug delivery apparatus for skin administration ,the drug delivery apparatus is controlled through temperature and pressure coupling and is used for controlling a drug to be administered in a manner of noninvasive skin penetration so as to achieve drug delivery treatment; the drug delivery apparatus is fixed to the skin; the drug delivery apparatus includes:
a pressure assembly and a temperature assembly, wherein the pressure assembly and the temperature assembly are used for controlling the pressure and temperature of skin administration to form a pressure difference of skin administration, and the pressure difference is the pressure difference of administration through the skin.

In some embodiments of this application, the drug delivery apparatus includes an auxiliary drug delivery assembly and a direct drug delivery assembly, wherein,
The pressure assembly includes a first pressure assembly and a second pressure assembly;
The auxiliary drug delivery assembly is provided with the first pressure assembly, the first pressure assembly generates first pressure, and the first pressure is pressure used for fixing the drug delivery apparatus to the skin and forming a closed drug delivery area;
The direct drug delivery assembly is provided with the second pressure assembly and the temperature assembly, the second pressure assembly generates second pressure, the temperature assembly performs temperature control in the closed drug delivery area, the first pressure, the second pressure and the temperature are controlled by the pressure difference of skin administration in the closed drug delivery area, and the pressure difference is the pressure difference of drug administration penetrating through the skin.

In some optional embodiments of the present application, the drug delivery apparatus further comprises a drug delivery control apparatus, wherein the drug delivery control apparatus includes :
a parameter determination module for determining an administration control parameter for skin administration, wherein the control parameter comprises a first control parameter and a second control parameter, a first parameter in the first control parameter and the second control parameter is a pressure control parameter, and the other parameter of the first control parameter and the second control parameter is a temperature control parameter; and
a pressure difference module which is used for controlling the pressure difference of skin administration through the pressure control parameter and the temperature control parameter, wherein the pressure difference is the pressure difference for feeding the skin through the skin.

According to the fourth aspect of the invention, a computer readable storage medium is provided, the computer readable storage medium stores computer instructions, and the computer instructions are used for enabling the computer to execute the drug delivery control method for skin administration.

The technical solution provided in the embodiments of the present application may include the following beneficial effects:
In the present application, through temperature and pressure coupling, a drug is controlled to be administered in a manner of non-invasive skin penetration so as to achieve drug administration treatment. By determining a drug administration control parameter for skin administration, the administration control parameter includes a pressure control parameter and a temperature control parameter, and the pressure difference of administration at the skin administration position is controlled through the coupling of the pressure control parameter and the temperature control parameter, so as to form a pressure difference for the administration of the penetrating skin. The pressure difference of non-invasive skin penetrating of the drug is formed by setting the pressure and the temperature together to form the skin drug delivery part, the problem that in the prior art, the drug administration efficiency is low is solved, and the technical effect of improving the drug delivery efficiency is achieved.

### BRIEF DESCRIPTION OF THE DRAWINGS

The drawings constituting a part of the present application are used to provide a further understanding of the present application, so that other features, objectives and advantages of the present application become more obvious. The drawings of the illustrative embodiments of the present application and the description thereof are used to explain the present application, and do not constitute an improper limitation on the present application. In the drawings:
FIG. 1 is a flow chart of a drug delivery control method for skin administration provided by the present application;
FIG. 2 is a flow chart of a drug delivery control method for skin administration provided by the present application;
FIG. 3 is a flow chart of a drug delivery control method for skin administration provided by the present application;
FIG. 4 is a flow chart of a drug delivery control method for skin administration provided by the present application;
FIG. 5 is a structural diagram of a drug delivery control apparatus for skin administration provided by the present application;
FIG. 6 is a structural diagram of another administration control apparatus for skin administration provided by the present application; and
FIG. 7 and FIG. 8 are structural diagrams of a drug delivery apparatus for skin administration according to the present application.

### DETAILED IMPLEMENTATION OF THE DISCLOSURE

In order to enable a person skilled in the art to better understand the solutions of the present application, the technical solutions in the embodiments of the present application will be clearly and completely described below with reference to the accompanying drawings in the embodiments of the present application. All other embodiments obtained by a person of ordinary skill in the art based on the embodiments of the present application without creative efforts shall fall within the protection scope of the present application.

It should be noted that the terms " first ", " second ", and the like in the specification, claims, and accompanying drawings of the present application are used to distinguish similar objects, and do not need to be used to describe a specific sequence or sequence. It should be understood that the data used in this way can be interchanged under appropriate circumstances so as to facilitate the embodiments of the present application described herein. In addition, the terms " include " and " have " and any deformation thereof are intended to cover non-exclusive inclusion, for example, a process, method, system, product, or apparatus that includes a series of steps or units is not necessarily limited to those steps or units that are clearly listed, but may include other steps or units that are not clearly listed or inherent to these processes, methods, products, or apparatuss.

In the present application, the terms " upper ", " lower ", " left ", " right ", " front ", " rear ", " top ", " bottom ", " inner ", " outside ", " middle ", " vertical ", " horizontal ", " transverse ", " longitudinal " and the like are oriented or positional relationships based on the orientation or positional relationship shown in the drawings. These terms are primarily intended to better describe the present application and the embodiments thereof, and are not intended to limit the indicated apparatus, element, or component to have a particular orientation, or be constructed and operated in a particular orientation.

And, in addition to being used for representing an orientation or a positional relationship, the above-mentioned partial terms may also be used to represent other meanings, for example, the term " upper " may also be used to represent a certain attachment relationship or a connection relationship in some cases. For a person of ordinary skill in the art, the specific meanings of these terms in the present application can be understood according to specific situations.

In addition, the terms " mounted ", " disposed ", " provided ", " connected ", " connected ", and " sleeving " should be understood in a broad sense. For example, " connected " may be a fixed connection, a detachable connection, or an integral structure; may be a mechanical connection or an electrical connection; may be directly connected, or indirectly connected by means of an intermediate medium, or may be an internal communication between two apparatuss, elements or components. For a person of ordinary skill in the art, the specific meanings of the above terms in the present application can be understood according to specific situations.

The drug delivery control method is applied to a drug delivery apparatus provided with a temperature control module and a pressure control module. The drug delivery apparatus is connected with the skin drug delivery position. The temperature control module and the pressure control module are used for controlling the pressure and temperature in the drug delivery area formed by the drug delivery apparatus and the skin to form a drug penetrating skin drug delivery pressure difference, so that the drug penetrates through the skin for administration. In the embodiment of the invention, the pressure difference is formed in the drug administration area, the pressure difference provides power for the drug molecule to penetrate through the skin, and the drug molecule is formed through the pressure difference to reach the power of the direct drug delivery position after penetrating the skin. For example, the drug component enters the body through the drug delivery area on the back, a pressure difference is formed in the drug delivery area, the pressure difference provides power for the drug component to penetrate through the back skin, and a power component is provided to reach the power of the lung after penetrating the skin.

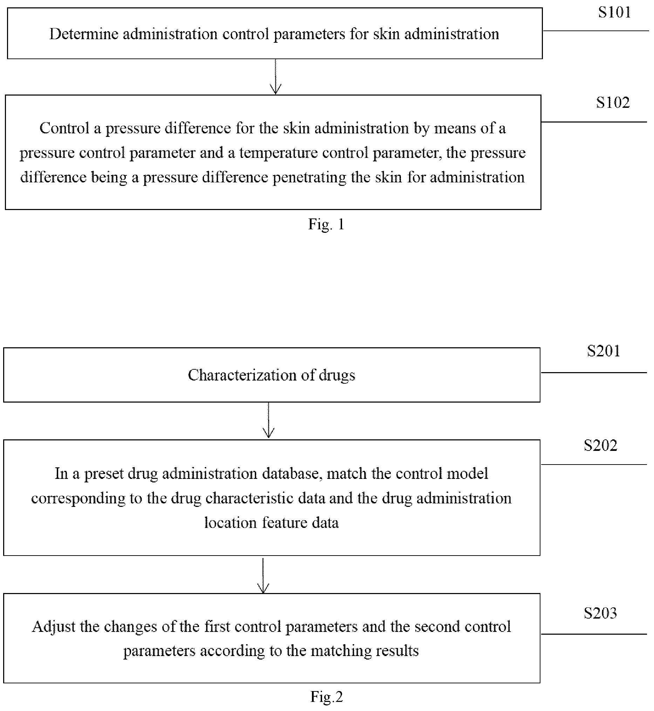

FIG. 1 is a flow chart of a drug administration control method for skin administration according to the present application, and as shown in FIG. 1, the method includes the following steps:
S101: determining administration control parameters for skin administration;

The control parameter includes a first control parameter and a second control parameter, one of the first control parameter and the second control parameter is a pressure control parameter, and the other one of the first control parameter and the second control parameter is a temperature control parameter.

FIG. 2 is a flowchart of a drug administration control method for skin administration according to the present application, as shown in FIG. 2, the method includes the following steps:
S201: determining administration characteristic data;
The drug delivery feature data includes drug feature data and drug delivery position feature data, the drug feature data is feature data used for representing drug delivery drugs, and the drug feature data includes data used for representing drug components and drug structures, for example, for example, the components of the drug delivery drugs can be single-component or multi-component drugs, and the drug delivery drugs can be solid drugs, liquid drugs or gas drugs; the administration position characteristic data is used for representing the position of administration treatment, and the administration position characteristic data includes a drug administration skin position for direct contact with the drug delivery apparatus and a position corresponding to a condition for drug delivery treatment target action, for example, for example, the drug delivery skin position in direct contact with the drug delivery apparatus can be a back lung acupoint location, and the drug delivery skin position in direct contact with the drug delivery apparatus can be a facial position; the position corresponding to the disease treatment target action can be the lung position corresponding to the lung disease, and the position corresponding to the disease treatment target effect can be the face skin layer or the face dermis layer position.

S202, matching a drug delivery control model corresponding to the drug feature data and the drug delivery position feature data in a preset drug delivery database;
The drug delivery control model is a model used for representing the change of the first control parameter and the second control parameter in the administration time. The drug delivery control model is a model for controlling the pressure and temperature in the drug delivery area to change over time within a preset drug delivery time period, and is used for guiding the drug delivery temperature and drug delivery pressure corresponding to different drugs and different drug delivery positions to be different. For example, during drug delivery treatment of lung diseases, component drug A is administered on the back, the first pressure is VA 1 min-VA 1 max, the second pressure is VA 2 min-VA 2 max, the temperature T 1 min-T 1 max, and on the back. A closed drug delivery area is formed, the pressure difference in the closed drug delivery area is ΔVAmin-ΔVAmax, and when the face is beautified, The first pressure is VB1min-VB1max, the second pressure is VB2min-VB2max, the temperature T2min-T2max forms a closed drug delivery area on the back, and the pressure difference in the closed drug delivery area is ΔVBmin-ΔVBmax. The drug delivery temperature and the drug delivery pressure corresponding to different drugs and different drug delivery positions are different according to different skin tolerance degrees of the back skin and the facial skin, and the drug administration temperature and the drug delivery pressure at the corresponding drug delivery position are adjusted according to different drugs and without drug delivery positions.

S203: determining the change of the first control parameter and the second control parameter according to the drug delivery control model.

Controlling the first control parameter and the second control parameter according to the drug delivery control model, and controlling the temperature parameter and the pressure parameter in the drug delivery process to control the drug delivery pressure difference.

In an optional embodiment of the present application, a method for controlling in a drug delivery process is provided. The method includes the following steps: acquiring a drug delivery instruction, identifying a drug delivery instruction to obtain drug delivery feature data, matching a drug delivery control model corresponding to the drug delivery feature data in a preset drug delivery database, controlling the drug delivery apparatus to change the pressure and temperature according to the drug delivery control model, and detecting the pressure difference in the drug delivery area through a pressure difference detection element provided in the drug delivery apparatus, and if not, the drug delivery control model generates pressure adjustment parameter and temperature adjustment parameter according to the currently detected pressure difference data, the pressure module performs pressure adjustment according to the pressure adjustment parameter, the temperature module performs temperature adjustment according to the temperature adjustment parameter, feedback adjustment is carried out on the basis of pressure difference data obtained through monitoring according to the drug delivery control model, temperature difference data in the drug delivery area is controlled to be within a preset drug delivery pressure difference range, and skin administration is carried out.

In the embodiment of the invention, feedback control is carried out in the drug delivery process by arranging a pressure difference detection element in the drug delivery apparatus, the detected pressure difference data is compared with a preset drug delivery pressure difference range, temperature and pressure control is achieved in the drug delivery process, then control over the drug delivery pressure difference is achieved, the pressure difference forms power of the drug penetrating through the skin to act on the disease position, and the drug delivery efficiency is improved.

S102, the pressure difference of skin administration is controlled through the pressure control parameters and the temperature control parameters, and the pressure difference is the pressure difference of administration through the skin.

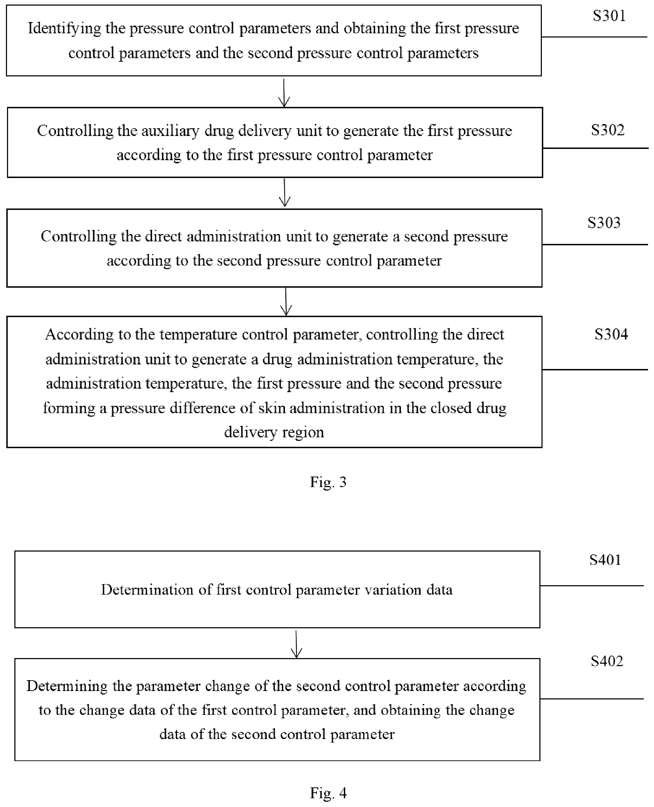

In another optional embodiment of the present application, a drug delivery control method for skin administration is provided. The drug delivery control method is applied to a drug delivery apparatus provided with an auxiliary drug delivery unit and a direct drug delivery unit, and FIG. 3 is a flowchart of a drug delivery control method for skin administration provided by the present application. As shown in FIG. 2, the method includes the following steps:
S301: identifying a pressure control parameter to obtain a first pressure control parameter and a second pressure control parameter;
Both the auxiliary drug delivery unit and the direct drug delivery unit are equipped with pressure modules, which require pressure control parameters to regulate the pressure, the pressure control parameters in the first pressure control parameter and the second pressure control parameter are pressure control parameters used for controlling the auxiliary drug delivery unit, and the other pressure control parameters in the first pressure control parameter and the second pressure control parameter are pressure control parameters used for controlling the direct drug delivery unit.

S302, controlling an auxiliary drug delivery unit to generate a first pressure according to the first pressure control parameter, wherein the first pressure is used for fixing the administration apparatus to the skin and forming a closed administration area;
The first pressure control parameter is a parameter for controlling the pressure of the auxiliary drug delivery unit. The pressure apparatus is arranged in the auxiliary drug delivery unit, for example, air is pumped through the air extraction assembly of the auxiliary drug delivery unit, the drug delivery apparatus is fixed to the skin administration position through air extraction treatment, and a closed drug delivery area is formed at the administration position.

S303, controlling the direct drug delivery unit to generate a second pressure according to the second pressure control parameter, wherein the first administration pressure is less than the second administration pressure;
The second pressure control parameter is a parameter for controlling the pressure change of the direct drug delivery unit. Through the pressure apparatus arranged in the direct drug delivery unit, for example, the inflation assembly in the direct drug delivery unit is inflated, the second pressure is generated through inflation treatment, the first pressure is set to be smaller than the second pressure, the drug delivery apparatus and the skin are fixed through the first pressure, the drug delivery apparatus forms a closed drug delivery area on the skin, the pressure difference is formed at the closed drug delivery area through the difference value of the first pressure and the second pressure, and the pressure difference is provided for the drug to penetrate through the skin.

In another alternative embodiment of the present application, the drug loading module is disposed in the direct drug delivery unit, and if the drug delivery drug is a solid or liquid structure, the solid drug or the liquid drug is disposed on the drug loading module in the direct drug delivery unit, the pressure module in the direct drug delivery unit is controlled to generate a second pressure, and the first pressure formed by the auxiliary drug delivery unit and the second pressure and the drug delivery temperature formed directly to the drug delivery unit form a drug delivery pressure difference, and the solid drug or the liquid drug component penetrates through the skin for administration; and if the drug delivery drug is of a gas structure, a second pressure is generated by a drug gas for drug administration and a pressure module through inflation control pressure, a drug delivery pressure difference is formed by assisting the first pressure formed by the drug delivery unit and the second pressure and the drug delivery temperature formed by the drug delivery drug unit, and the drug component penetrates through the skin for administration.

S304: according to the temperature control parameter, controlling the direct drug delivery unit to generate a drug administration temperature, the administration temperature, the first pressure and the second pressure forming a pressure difference of skin administration in the closed drug delivery area.

A temperature control module, such as a heating assembly, is arranged in the direct drug delivery unit, the temperature of the closed drug delivery area is controlled to rise, and the drug delivery pressure difference of the closed drug delivery area is formed under the combined action of the heating assembly, the first pressure and the second pressure.

In another optional embodiment of the present application, a method for forming a pressure difference of skin administration in a closed drug delivery area is provided,
Obtaining drug delivery pressure difference data, wherein the drug delivery pressure difference data is data used for representing the pressure difference in the closed drug delivery area;
Whether the drug delivery pressure difference data satisfies a preset drug delivery pressure difference range is judged,
If not, generating an updated pressure control parameter, wherein the updated first pressure data and the updated second pressure data are determined according to the updated pressure control parameter until the administration pressure difference data satisfies a preset drug delivery pressure difference range;
If so, the drug is administered in a closed drug delivery area.

In another optional embodiment of the present application, a method for controlling a drug delivery pressure difference in a closed drug delivery area through temperature control is also provided, the method including:
Obtaining drug delivery pressure difference data, wherein the drug delivery pressure difference data is data used for representing a pressure difference in a closed area;
Determining whether the drug delivery pressure difference data satisfies a preset drug delivery pressure difference range;
If not, generating an update temperature control parameter, wherein the update temperature is determined according to the temperature control parameter until the drug delivery pressure difference satisfies a preset drug delivery pressure difference range;
If so, administration is carried out in the sealed administration area.

In the embodiment of the invention, the pressure difference in the drug delivery area is detected through the pressure difference detection element arranged in the drug delivery apparatus, whether the pressure difference in the drug delivery area satisfies the preset drug delivery pressure difference range or not is detected, for example, when the drug delivery treatment of the renal disease is carried out, the drug delivery pressure difference range at the drug delivery position corresponds to the kidney disease treatment rule, and when the drug delivery treatment of the lung disease is carried out, the drug delivery pressure range at the drug delivery position corresponds to the lung disease treatment rule. In the range of the drug delivery pressure difference, the drug component penetrates through the skin to arrive at the drug delivery part, and when it is detected that the drug delivery pressure difference does not satisfy the pressure difference data that the drug can penetrate through the skin and enter the lesion, the drug delivery control parameter in the drug delivery apparatus is controlled to change to form the effect that the drug penetrates through the skin to reach the lesion for drug delivery treatment.

The invention further provides a method for controlling the temperature. The method includes the following steps: detecting the temperature in the drug delivery area through a temperature detection element arranged in the drug delivery apparatus, judging whether the detection temperature is within a preset temperature range, if the detection temperature does not satisfy the preset temperature range, adjusting the pressure difference in the closed drug delivery area through adjustment of the temperature within a preset temperature range, adjusting and controlling the pressure difference of the closed drug delivery area through temperature adjustment through adjustment of the temperature within a preset temperature range, and avoiding the situation that the skin in the drug delivery position is injured in the drug delivery process.

In another optional embodiment of the present application, a drug delivery control method for skin administration is provided. When skin administration is performed, a first pressure in a direct drug delivery unit is set as va, a second pressure of the auxiliary drug delivery unit is vb, a drug administration temperature of the direct drug delivery unit is T, a pressure difference Ci, a C drug delivery pressure difference and a first pressure are formed in the drug delivery area when performing control to perform skin administration, ci = f (va, vb, T), and when skin administration is performed, the drug delivery pressure difference is jointly determined through a first pressure, a second pressure, and a drug administration temperature.

FIG. 4 is a flow chart of a drug delivery control method for skin administration provided by the present application, as shown in FIG. 4, The method includes the following steps:
One of the first control parameter and the second control parameter is a pressure control parameter, and the other one of the first control parameter and the second control parameter is a temperature control parameter. Through joint cooperation of the pressure control parameter and the temperature control parameter, the pressure difference of the closed drug delivery area is controlled.

S401: determining first control parameter change data;
The first control parameter change data being parameter change data used for representing a first control parameter;
S402: determining a parameter change of the second control parameter according to the first control parameter change data to obtain second control parameter change data.

When the pressure difference in the closed drug delivery area is controlled, the pressure difference is controlled through the first control parameter and the second control parameter, and the temperature control parameter and the pressure control parameter are coupled and controlled to jointly control the drug delivery pressure difference. When the first control parameter and the second control parameter are adjusted, the first control parameter and the second control parameter influence each other, the change of the second control parameter is determined according to the change of the first control parameter, the pressure difference of the closed drug delivery area is controlled, and dynamic control of the pressure difference in the drug delivery process is achieved.

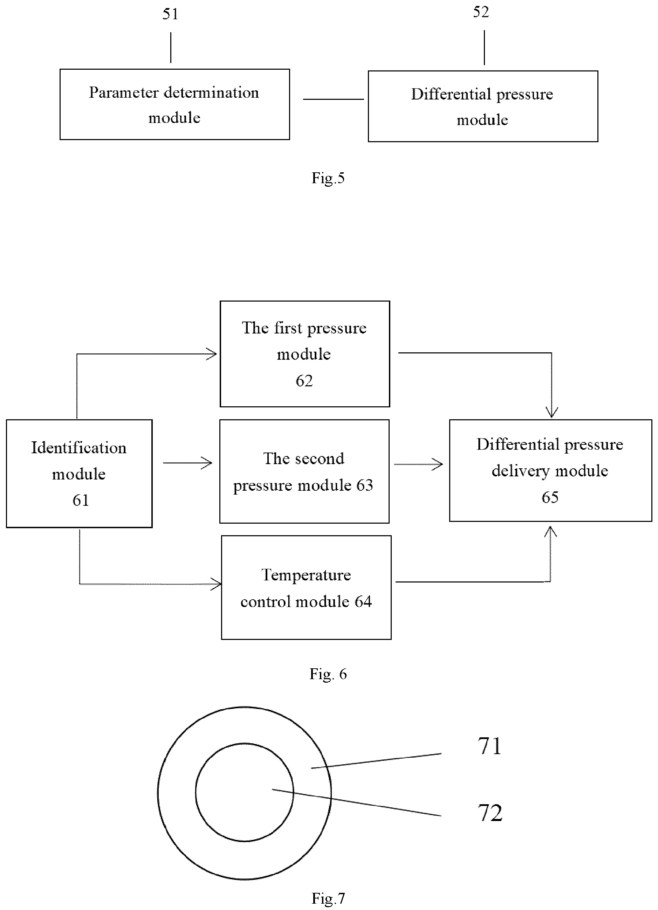

FIG. 5 is a structural diagram of an administration control apparatus for skin administration according to the present application, as shown in FIG. 5, the administration control apparatus includes:
A parameter determination module 51, which is used for determining an administration control parameter for skin administration, wherein the control parameter includes a first control parameter and a second control parameter, a first parameter in the first control parameter and the second control parameter is a pressure control parameter, and the other parameter in the first control parameter and the second control parameter is a temperature control parameter;
Pressure difference module 52 is used for controlling the pressure difference of skin administration through pressure control parameter and temperature control parameter, and the pressure difference is the pressure difference of administration of penetrating skin.

FIG. 6 is a structural diagram of another administration control apparatus for skin administration according to the present application, as shown in FIG. 6, the apparatus includes:
An identification module 61, used for identifying a pressure control parameter to obtain a first pressure control parameter and a second pressure control parameter;
The first pressure module 62 is used for controlling the auxiliary drug delivery unit to generate a first pressure according to the first pressure control parameter, and the first pressure is used for fixing the drug delivery apparatus to the skin and forming a closed drug delivery area;
A second pressure module 63, used for controlling, according to a second pressure control parameter, a direct drug delivery unit to generate a second pressure, the first pressure being less than the second pressure;
A temperature control module 64, used for controlling, according to the temperature control parameter, the direct drug delivery unit to generate a drug administration temperature;
A pressure difference drug delivery module 65 is used for forming a skin drug delivery pressure difference in the sealed drug delivery area according to the drug delivery temperature, the first pressure and the second pressure.

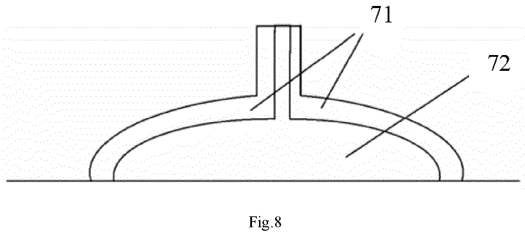

In another optional embodiment of the present application, FIG. 7 and FIG. 8 are structural diagrams of a drug delivery apparatus for skin administration provided by the present application The drug delivery apparatus is controlled through pressure and temperature coupling and is used for controlling a drug to be administered in a manner of non-invasive skin penetration so as to achieve drug delivery treatment The drug delivery apparatus includes:
A pressure assembly and a temperature assembly the pressure assembly and the temperature assembly control the pressure and temperature of skin administration to form a pressure difference of skin administration, and the pressure difference is the pressure difference of administration of penetrating skin.

The drug delivery apparatus includes an auxiliary drug delivery assembly 71 and a direct drug delivery assembly 72,
The pressure assembly includes a first pressure assembly and a second pressure assembly;
An auxiliary drug delivery assembly is provided with a first pressure assembly, the first pressure assembly generates a first pressure, and the first pressure is pressure used for fixing the drug delivery apparatus to the skin and forming a closed drug delivery area;
A direct drug delivery assembly is provided with a second pressure assembly and a temperature assembly, the second pressure assembly generates second pressure, the temperature assembly performs temperature control in the closed drug delivery area, the first pressure, the second pressure and the temperature are controlled by the pressure difference of skin administration in the closed drug delivery area, and the pressure difference is the pressure difference of administration through the skin.

The first pressure assembly can be used for absorbing gas in the auxiliary drug delivery assembly and is used for forming the first pressure is used for fixing the administration apparatus with the skin , the second pressure assembly can be an inflation apparatus and is used for forming a second pressure in the direct drug delivery assembly, the direct drug delivery assembly and the skin form a closed drug delivery area, a heating module is arranged in the direct drug delivery assembly and used for heating in the closed drug delivery area, and a pressure difference used for penetrating the drug component into the skin is formed in the closed drug delivery area.

The specific manner in which the execution operations of the units in the above embodiments have been described in detail in the embodiments related to the method, which will not be elaborated herein.

In conclusion, in the present application, through temperature and pressure coupling, a drug is controlled to be administered in a manner of non-invasive skin penetration so as to achieve drug administration treatment. Through determining a drug administration control parameter for skin administration, the administration control parameter includes a pressure control parameter and a temperature control parameter, and the pressure difference of administration at the skin administration position is controlled through the coupling of the pressure control parameter and the temperature control parameter, so as to form a pressure difference for the administration of the penetrating skin. The pressure difference of non-invasive skin penetrating of the drug is formed by setting the pressure and the temperature together to form the skin drug delivery part, the problem that in the prior art, the drug administration efficiency is low is solved, and the technical effect of improving the drug delivery efficiency is achieved.

It should be noted that the steps shown in the flowcharts of the accompanying drawings may be executed in a computer system such as a group of computer-executable instructions, and although the logic sequence is shown in the flowcharts, in some cases, the steps shown or described may be executed in a different order.

It should be understood by those skilled in the art that the units or steps of the present application may be implemented by using a general-purpose computing apparatus, which may be concentrated on a single computing apparatus or distributed on a network composed of a plurality of computing apparatuss, and optionally, they may be implemented by using program codes executable by a computing apparatus, so that they may be stored in a storage apparatus to be executed by a computing apparatus, or they are manufactured into individual integrated circuit modules respectively, or a plurality of modules or steps in the integrated circuit modules may be manufactured into a single integrated circuit module. In this way, the present application is not limited to any specific combination of hardware and software.

The above are only preferred embodiments of the present application and are not intended to limit the present application, and for a person skilled in the art, the present application may have various modifications and changes. Any modification, equivalent replacement, improvement and the like made within the spirit and principle of the present application should be included in the protection scope of the present application..

## Claims

1. A drug delivery control method for skin administration, **characterized in that**, through temperature and pressure coupling control, the drug delivery control method is used for controlling a drug to be administered in a manner of non-invasive skin penetration so as to achieve drug delivery treatment, and the drug delivery control method comprises:
determining an administration control parameter for skin administration, wherein the control parameter comprises a first control parameter and a second control parameter, one of the first control parameter and the second control parameter is a pressure control parameter, and the other one of the first control parameter and the second control parameter is a temperature control parameter; and
controlling a pressure difference of the skin administration through the pressure control parameter and the temperature control parameter, wherein the pressure difference is the pressure difference of the administration of the skin through the skin.

2. The drug delivery control method according to claim 1, **characterized in that** the drug delivery control method is applied to a drug delivery apparatus provided with an auxiliary drug delivery unit and a direct drug delivery unit, and the drug delivery control method comprises:
identifying the pressure control parameter to obtain a first pressure control parameter and a second pressure control parameter;
controlling the auxiliary drug delivery unit to generate a first pressure according to the first pressure control parameter, wherein the first pressure is used for fixing the drug delivery apparatus to the skin and forming a closed drug delivery area;
controlling the direct drug delivery unit to generate a second pressure according to the second pressure control parameter, wherein the first pressure is less than the second pressure; and
controlling the direct drug delivery unit to generate an administration temperature according to the temperature control parameter, wherein the administration temperature, the first pressure and the second pressure form a pressure difference of the skin administration in the closed drug delivery area.

3. The drug delivery control method according to claim 2, **characterized in that** the drug delivery temperature, the first pressure and the second pressure form the pressure difference of the skin administration in the closed drug delivery area, comprising:
obtaining drug delivery pressure difference data, wherein the drug delivery pressure difference data is data used for representing the pressure difference in the closed drug delivery area;
determining whether the drug delivery pressure difference data satisfies a preset drug delivery pressure difference range;
if not, generating an updated pressure control parameter, wherein the updated first pressure data and the updated second pressure data are determined according to the updated pressure control parameter until the administration pressure difference data satisfies the preset drug delivery pressure difference range; and
if so, carrying out administration in the closed drug delivery area.

4. The drug delivery control method according to claim 2, **characterized in that** the drug delivery temperature, the first pressure and the second pressure form the pressure difference of the skin administration in the closed drug delivery area, comprising:
obtaining drug delivery pressure difference data, wherein the drug delivery pressure difference data is data used for representing a pressure difference in a closed area;
determining whether the drug delivery pressure difference data satisfies a preset drug delivery pressure difference range;
if not, generating an update temperature control parameter, wherein the update temperature is determined according to the temperature control parameter until the drug delivery pressure difference satisfies a preset drug delivery pressure difference range; and
if so, carrying out administration in the closed drug delivery area.

5. The drug delivery control method according to claim 1, **characterized in that** determining the administration control parameter for skin administration comprises:
determining first control parameter change data, wherein the first control parameter change data is parameter change data used for representing a first control parameter; and
determining a parameter change of the second control parameter according to the first control parameter change data to obtain second control parameter change data.

6. The drug delivery control method according to claim 1, **characterized in that** determining administration control parameter for skin administration comprises:
determining administration feature data, wherein the administration feature data comprises drug feature data and administration position feature data;
matching a drug delivery control model corresponding to the drug feature data and the administration position feature data in a preset drug delivery database, wherein the drug delivery control model is a model used for representing a change of the first control parameter and the second control parameter in a drug delivery time; and
determining the change in the first control parameter and the second control parameter according to the drug delivery control model.

7. The drug delivery control method according to claim 1, **characterized in that** controlling the pressure difference of skin administration through the pressure control parameter and the temperature control parameter comprises:
determining the pressure difference of the skin administration according to the first pressure, the second pressure and the administration temperature, wherein the expression of the administration pressure difference is: ci = f (va, vb, T), wherein ci is the pressure difference, va is the first pressure in the direct drug delivery unit, vb is the second pressure of the auxiliary drug delivery unit, and T is the administration temperature of the direct drug delivery unit.

8. The drug delivery control method according to claim 1, **characterized in that** a temperature detection element is arranged in the drug delivery apparatus and is used for detecting the temperature in the drug delivery area so as to control matching drug delivery, and the drug delivery control method comprises the following steps:
acquiring administration temperature detection data, wherein the administration temperature detection data is temperature data in an administration area;
determining the administration detection data on the basis of a preset temperature range; and
if the detection temperature does not satisfy the preset temperature range, generating temperature control parameter.

9. A drug delivery control apparatus for skin administration, wherein, through temperature and pressure coupling control, the drug delivery control apparatus is used for controlling a drug to be administered in a manner of non-invasive skin penetration so as to achieve drug delivery treatment, and the drug delivery control apparatus comprises:
a parameter determination module, used for determining an administration control parameter for skin administration, wherein the control parameter comprises a first control parameter and a second control parameter, a first parameter in the first control parameter and the second control parameter is a pressure control parameter, and the other parameter of the first control parameter and the second control parameter is a temperature control parameter; and
a pressure difference module, used for controlling a pressure difference of the skin administration through the pressure control parameter and the temperature control parameter, wherein the pressure difference is the pressure difference of the administration of the skin through the skin.

10. The drug delivery control apparatus according to claim 9, **characterized in that** the drug delivery control apparatus is applied to a drug delivery apparatus provided with an auxiliary drug delivery unit and a direct drug delivery unit, and the drug delivery control apparatus comprises:
an identification module, used for identifying the pressure control parameter to obtain a first pressure control parameter and a second pressure control parameter;
a first pressure module, used for controlling the auxiliary drug delivery unit to generate a first pressure according to the first pressure control parameter, wherein the first pressure is used for fixing the drug delivery apparatus to the skin and forming a closed drug delivery area;
a second pressure module, used for controlling the direct drug delivery unit to generate a second pressure according to the second pressure control parameter, wherein the first pressure is less than the second pressure;
a temperature control module, used for controlling the direct drug delivery unit to generate a drug delivery temperature according to the temperature control parameter; and
a pressure difference drug delivery module, used for forming the pressure difference of the skin administration in the closed drug delivery area according to the drug delivery temperature, the first pressure and the second pressure.

11. The drug delivery control apparatus according to claim 10, **characterized in that** the pressure difference drug delivery module comprises:
a pressure difference acquisition module, used for acquiring drug delivery pressure difference data, wherein the drug delivery pressure difference data is data used for representing the pressure difference in the closed drug delivery area;
a pressure difference judgment module, used for judging whether the drug delivery pressure difference data satisfies a preset drug delivery pressure difference range or not;
a pressure difference control module, used for: if not, generating an updated pressure control parameter, wherein the updated first pressure data and the updated second pressure data are determined according to the updated pressure control parameter until the numerical value of the drug delivery pressure difference data satisfies the preset drug delivery pressure difference range; and
if the numerical value of the drug delivery pressure difference data satisfies the preset drug delivery pressure difference range, performing administration in the closed drug delivery area.

12. The drug delivery control apparatus according to claim 10, wherein the pressure difference drug delivery module comprises:
a pressure difference acquisition module, used for acquiring drug delivery pressure difference data,
wherein the drug delivery pressure difference data is data used for representing the pressure difference in the closed drug delivery area;
a pressure difference judgment module, used for judging whether the drug delivery pressure difference data satisfies a preset drug delivery pressure difference range or not;
a temperature control module, used for: if not, generating an update temperature control parameter, wherein the update temperature is determined according to the temperature control parameter until the drug delivery pressure difference satisfies a preset drug delivery pressure difference range; and if so, carrying out administration in the closed drug delivery area.

13. The administration control apparatus according to claim 9, **characterized in that** the parameter determination module comprises:
a first control parameter module, used for determining first control parameter change data, wherein the first control parameter change data is parameter change data used for representing a first control parameter; and
a second control parameter module, used for determining a parameter change of the second control parameter according to the first control parameter change data, so as to obtain second control parameter change data.

14. The administration control apparatus according to claim 9, **characterized in that** the parameter determination module comprises:
a drug delivery data acquisition module, used for determining drug delivery feature data, wherein the drug delivery feature data comprises drug feature data and drug delivery position feature data;
a model matching module, used for matching a drug delivery control model corresponding to the drug feature data and the drug delivery position feature data in a preset drug delivery database, wherein the drug delivery control model is a model used for representing the change of the first control parameter and the second control parameter in the drug delivery time; and
an administration parameter determination module, used for determining the change of the first control parameter and the second control parameter according to the drug delivery control model.

15. The drug delivery control apparatus according to claim 9, **characterized in that**,
the pressure difference module is used for determining the pressure difference of skin administration according to the first pressure, the second pressure and the administration temperature, wherein the expression of the administration pressure difference is ci = f (va, vb, T), ci is the pressure difference, va is the first pressure in the direct drug delivery unit, vb is the second pressure of the auxiliary drug delivery unit, and T is the administration temperature of the direct drug delivery unit.

16. The drug delivery control apparatus according to claim 9, **characterized in that** a temperature detection element is arranged in the drug delivery apparatus and is used for detecting the temperature in the drug delivery area so as to control matching drug delivery, and the drug delivery control apparatus comprises:
a drug delivery temperature detection module, used for obtaining drug administration temperature detection data, wherein the drug delivery temperature detection data is temperature data in a drug delivery area;
a drug delivery temperature judgment module, used for judging the administration detection data based on a preset temperature range; and
a drug delivery temperature control module, used for generating a temperature control parameter if the detection temperature does not satisfy a preset temperature range.

17. A drug delivery apparatus for skin administration, **characterized in that**, through temperature and pressure coupling control, the drug delivery apparatus is used for controlling drug administration in a manner of non-invasive skin penetration so as to achieve drug delivery treatment, the drug delivery apparatus is fixed to the skin, and the drug delivery apparatus comprises:
a pressure assembly and a temperature assembly, wherein the pressure assembly and the temperature assembly are used for controlling the pressure and temperature of skin administration to form a pressure difference of skin administration, and the pressure difference is the pressure difference of administration of the skin through the skin.

18. The drug delivery apparatus according to claim 17, **characterized in that** the drug delivery apparatus comprises an auxiliary drug delivery assembly and a direct drug delivery assembly, wherein
the pressure assembly comprises a first pressure assembly and a second pressure assembly;
the auxiliary drug delivery assembly is provided with the first pressure assembly, the first pressure assembly generates a first pressure, and the first pressure is pressure used for fixing the drug delivery apparatus to the skin and forming a closed drug delivery area; and
the direct drug delivery assembly is provided with the second pressure assembly and the temperature assembly, the second pressure assembly generates a second pressure, the temperature assembly performs temperature control in the closed drug delivery area, the first pressure, the second pressure and the temperature are controlled by the pressure difference of the skin administration in the closed drug delivery area, and the pressure difference is the pressure difference of drug administration penetrating through the skin.

19. The drug delivery apparatus according to claim 17, **characterized in that** the drug delivery apparatus further comprises a drug delivery control apparatus, and the drug delivery control apparatus comprises:
a parameter determination module, used for determining an administration control parameter for skin administration, wherein the control parameter comprises a first control parameter and a second control parameter, a first parameter in the first control parameter and the second control parameter is a pressure control parameter, and the other parameter of the first control parameter and the second control parameter is a temperature control parameter; and
a pressure difference module, used for controlling the pressure difference of skin administration through the pressure control parameter and the temperature control parameter, wherein the pressure difference is the pressure difference for feeding the skin through the skin.

20. A computer-readable storage medium, **characterized in that** the computer-readable storage medium stores a computer instruction, and the computer instruction is used for enabling a computer to execute the drug delivery control method for the skin administration according to any one of claims 1-8.
